(19) Europäisches Patentamt
European Patent Office
Office européen des brevets

(11) **EP 4 666 954 A1**

(12) **EUROPEAN PATENT APPLICATION**
published in accordance with Art. 153(4) EPC

(43) Date of publication:
**24.12.2025 Bulletin 2025/52**

(21) Application number: **24759472.4**

(22) Date of filing: **23.01.2024**

(51) International Patent Classification (IPC):
**A61B 6/06** (2006.01)          **A61B 6/03** (2006.01)

(52) Cooperative Patent Classification (CPC):
**A61B 6/00; A61B 6/03; A61B 6/06**

(86) International application number:
**PCT/CN2024/073625**

(87) International publication number:
**WO 2024/174794 (29.08.2024 Gazette 2024/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **21.02.2023 CN 202310144907**

(71) Applicants:
• **Midea Group (Shanghai) Co., Ltd.**
  **Shanghai 201702 (CN)**
• **Midea Group Co., Ltd.**
  **Foshan, Guangdong 528311 (CN)**

(72) Inventors:
• **LI, Aaron**
  **Shanghai 201702 (CN)**
• **MA, Jingwei**
  **Shanghai 201702 (CN)**

(74) Representative: **Ran, Handong**
  **Maucher Jenkins**
  **Seventh Floor Offices**
  **Artillery House**
  **11-19 Artillery Row**
  **London SW1P 1RT (GB)**

(54) **COLLIMATOR AND CT MACHINE**

(57)     Provided are a collimator and a CT machine. The collimator comprises a collimator main body (100), wherein the collimator main body (100) has a first collimating surface (131) facing or facing away from an X-ray tube, and a second collimating surface (141) facing or facing away from the X-ray tube; the curvature of the first collimating surface (131) is greater than that of the second collimating surface (141); the first collimating surface (131) and the second collimating surface (141) are arranged in a staggered manner; and a ray channel (150) penetrating the collimator is formed in the collimator main body (100), and the ray channel (150) is distributed in the first collimating surface (131) and the second collimating surface (141).

FIG. 1

**Description**

**CROSS-REFERENCE TO RELATED APPLICATIONS**

This application claims priority to Chinese Patent Application No. 202310144907.4, filed on February 21, 2023, the contents of which are herein incorporated by reference in their entireties.

**TECHNICAL FIELD**

The present disclosure relates to the technical field of medical device, and in particular to a collimator and a CT machine.

**BACKGROUND**

A primary function of a collimator of a CT machine may be to limit the amount of emitted X-rays through setting an emission width of the X-rays according to clinical scanning requirements. In order to enable a shape of a projection of the X-rays through the collimator onto a detector to be substantially a rectangle, the collimator may be configured to have an arc-shaped structure. However, such a configuration may result in the collimator occupying excessive space in a Y-axis direction of the CT machine, thereby resulting in limited space within the CT machine.

**SUMMARY**

Some embodiments of the present disclosure aim to address a technical problem of limited space within a current CT machine at least to a certain extent, and accordingly, a collimator and a CT machine may be provided.

In a first aspect, some embodiments of the present disclosure may provide a heat dissipation system. The heat dissipation system may include a collimator body. The collimator body may include a first collimating surface facing or facing away from an X-ray tube and a second collimating surface facing or facing away from the X-ray tube. A curvature of the first collimating surface may be greater than a curvature of the second collimating surface. The first collimating surface and the second collimating surface may be arranged in a staggered manner. The collimator body may define a ray channel penetrating through the collimator body. The ray channel may be defined by the first collimating surface and the second collimating surface.

In a second aspect, some embodiments of the present disclosure may provide a CT machine. The CT machine may include the above-mentioned collimator.

**BRIEF DESCRIPTION OF THE DRAWINGS**

In order to more clearly illustrate the technical solutions in some embodiments of the present disclosure, a brief introduction will be given below to the drawings required in the description of the embodiments. It is evident that the drawings described below are merely some embodiments of the present disclosure, and those skills in the art may obtain other drawings based on the following drawings without creative work.

　　FIG. 1 is a front schematic view of a collimator according to some embodiments of the present disclosure.
　　FIG. 2 is a top schematic view of the collimator according to some embodiments of the present disclosure.
　　FIG. 3 is a schematic view of a first collimating portion according to some embodiments of the present disclosure.

In the figures:
100, collimator body; 110, first collimating portion; 111, first sidewall; 112, second sidewall; 120, second collimating portion; 130, first collimating section; 131, first collimating surface; 132, third collimating surface; 140, second collimating section; 141, second collimating surface; 142, fourth collimating surface; 150, ray channel.

**DETAILED DESCRIPTION**

With reference to the accompanying drawings in some embodiments of the present disclosure, the technical solutions of some embodiments of the present disclosure will be clearly and completely described hereinafter. It is apparent that the described embodiments may merely be part of the embodiments of the present disclosure rather than all of the embodiments. Based on the embodiments of the present disclosure, all other embodiments obtained by those skilled in the art without inventive effort may fall within the protection scope of the present disclosure.

It should be noted that all directional indications in the embodiments of the present disclosure may only be used to explain relative positional relationships, movement conditions, etc., among respective components under a particular posture. Once the particular posture changes, the directional indications may be correspondingly changed.

In the present disclosure, unless otherwise expressly specified and defined, terms such as "connected" and "fixed" should be understood in a broad sense. For example, "fixed" may mean fixed connection, detachable connection, or integral formation; it may be a mechanical connection or an electrical connection; it may be directly connected or indirectly connected through an intermediate medium; it may refer to communication between two elements or to interaction between two elements, unless otherwise expressly limited. For those skilled in the art, the meanings of the above terms in the present disclosure may be understood according to particular circumstances.

In addition, in the present disclosure, the description involving "first," "second," etc. may be used merely for descriptive purposes and may not be understood as indicating or implying relative importance or implicitly indicating the number of the technical features. Accordingly, features defined as "first" or "second" may expressly or implicitly include at least one such feature. Moreover, the technical solutions among various embodiments may be combined with each other, provided that such combinations can be realized by those skilled in the art. However, when the technical solutions are mutually contradictory or technically infeasible, such combinations should be deemed not to exist and should not fall within the protection scope of the present disclosure.

Some technical solutions of the present disclosure will be described with reference to the drawings and embodiments hereinafter.

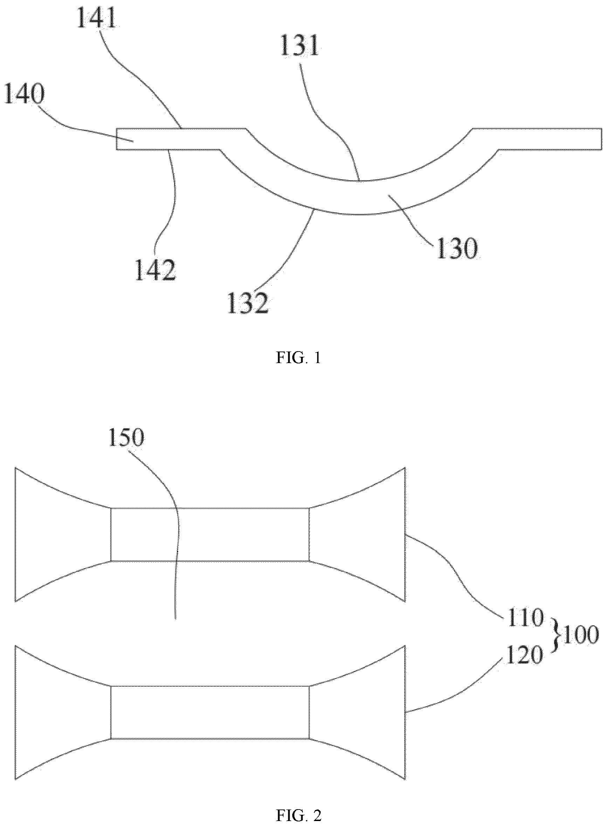

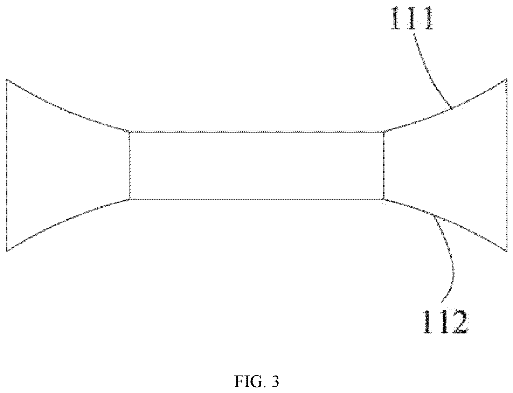

FIG. 1 is a front schematic view of a collimator according to some embodiments of the present disclosure. FIG. 2 is a top schematic view of the collimator according to some embodiments of the present disclosure. FIG. 3 is a schematic view of a first collimating portion according to some embodiments of the present disclosure.

As shown in FIGS. 1-3, some embodiments of the present disclosure may provide a collimator. The collimator may include a collimator body 100. The collimator body 100 may include a first collimating surface 131 facing or facing away from an X-ray tube and a second collimating surface 141 facing or facing away from the X-ray tube. A curvature of the first collimating surface 131 may be greater than a curvature of the second collimating surface 141. The first collimating surface 131 and the second collimating surface 141 may be arranged in a staggered manner. The collimator body 100 may define a ray channel penetrating through the collimator body 100. The ray channel 150 may be defined by the first collimating surface 131 and the second collimating surface 141. The collimator may be configured in a CT machine in order to save internal space of the CT machine.

In some embodiments, the collimator body 100 may be a fundamental part of the collimator of the present disclosure. The collimator body 100 may provide an installation base for at least some other parts of the collimator. The collimator body 100 may be configured as a main structure for constraining X-rays in the collimator. The collimator body 100 may be made of a tungsten material.

In some embodiments, in a case where the collimator is configured in the CT machine, the collimator may be disposed between the X-ray tube of the CT machine and a detector of the CT machine. In this way, a side of the collimator may face the X-ray tube and another side of the collimator may face away from the X-ray tube. In some embodiments, the collimator body 100 may include the first collimating surface 131 that is arranged on the side facing the X-ray tube or on the another side facing away from the X-ray tube and a second collimating surface 141 that is arranged on the side facing the X-ray tube or on the another side facing away from the X-ray tube. That is, the first collimating surface 131 of the collimator body 100 and the second collimating surface 141 of the collimator body 100 may be arranged on any one of the two opposite sides of the collimator body 100.

The collimator body 100 may define the ray channel 150. The ray channel 150 may be configured to penetrate through the collimator body 100. In a case where the collimator body 100 is disposed between the X-ray tube and the detector, the X-rays emitted from the X-ray tube may pass through the ray channel 150 of the collimator body 100 to the detector. Other parts of the collimator body 100 except the ray channel 150 may be configured to block unnecessary scattered rays and determine a beam coverage width of the X-rays during a single scan.

It should also be understood that the X-rays emitted from the X-ray tube may be in the form of a divergent beam centered at the focal point. In some embodiments, the X-rays may be fan-shaped. In this way, in order for the detector to better receive the X-rays emitted from the X-ray tube, the detector may be configured to have an arc-shaped structure such that the X-rays emitted from different parts of the X-ray tube may reach a surface of the detector substantially in perpendicular, thereby improving image quality of the X-rays onto the surface of the detector. In some embodiments, a shape of a projection of the X-rays, which is emitted from the X-ray tube and passes through the ray channel 150 of the collimator body 100, on the detector may be substantially a rectangle, thereby improving imaging quality of the CT machine and reducing additional radiation dose to the patient. In order to enable the shape of the projection of the X-rays, which is emitted from the X-ray tube and passes through the ray channel 150 of the collimator body 100, on the detector to be substantially a rectangle, the collimator body 100 may be configured to have an arc-shaped structure to constrain and correct the emitted X-rays from the X-ray tube.

In some embodiments, the first collimating surface 131 of the collimator body 100 may be configured to be a curved surface. At least a part of the ray channel 150 of the collimator body 100 may be defined by the first collimating surface 131, thereby allowing the collimator body 100 to at least partially be arc-shaped, further enabling a shape of the projection of the X-rays, which passes through the part of the ray channel 150 defined by the first collimating surface 131, on the detector to be relatively close to a rectangle. At least another part of the ray channel 150 of the collimator body 100 may be defined by the second collimating surface 141, enabling the X-rays to reach the detector after passing through the part of the ray channel 150 defined by the second collimating surface 141. The curvature of the second collimating surface 141 of the collimator body 100 may be smaller than the curvature of the first collimating surface 131, thereby enabling the second collimating surface 141 of the collimator body 100 to be flatter relative to the first collimating surface 131. In this way, the part of the collimator body 100 corresponding to the second collimating surface 141 may be flatter and occupy less space in a Y-axis direction of the CT machine than the part of the collimator body 100 corresponding to the first collimating surface 131, thereby reducing space occupied by the collimator in the CT machine and facilitating an internal layout of the CT machine.

It should be understood that since the part of the collimator body 100 corresponding to the second collimating surface 141 is flatter than the part of the collimator body 100 corresponding to the first collimating surface 131, a shape of the projection of the X-rays passing the second collimating surface 141 onto the detector to have a relatively large deviation from a rectangle. In this way, in a case where the collimator of some embodiments of the present disclosure is configured in the CT machine, the X-rays passing the first collimating surface 131 of the collimator body 100 may provide better imaging quality. During examination, a patient or test subject may be positioned such that important body parts to be examined, such as organs or the head, may face the X-rays passing the first collimating surface 131, thereby obtaining better imaging results of important parts of the test subject and enabling a medical staff to make accurate diagnosis on the important parts of the test subject. It should be understood that less important body parts of the test subject, such as limbs and etc., may have lower image quality requirements. In this way, the less important body parts of the test subject may face the X-rays passing the second collimating surface 141 to obtain a fairly clear diagnostic image, thereby enabling the medical staff to assess a general condition of the less important body parts of the test subject. In some embodiments, in a case where the limbs and etc. of the test subject show significant discomfort or require specialized examination, the test subject may position the limbs and etc. to correspond to or face the X-rays passing the first collimating surface 131, thereby obtaining clear images of the limbs of the test subject as well.

In the collimator of some embodiments of the present disclosure, the curvature of the first collimating surface 131 of the collimator body 100 may be different from the curvature of the second collimating surface 141 of the collimator body 100. The curvature of the first collimating surface 131 may be greater than the curvature of the second collimating surface 141, which allows the part of the collimator body 100 corresponding to the second collimating surface 141 to occupy less space than the part of the collimator body 100 corresponding to the first collimating surface 131, thereby enabling the collimator to have a relatively compact structure. Accordingly, in a case where the collimator of some embodiments of the present disclosure is configured in the CT machine, the space occupied by the collimator in the CT machine may be reduced. The shape of the projection of the X-rays passing the part of the ray channel 150 corresponding to or defined by the first collimating surface 131 onto the detector may be more similar to a rectangle than the shape of the projection of the X-rays passing the part of the ray channel 150 corresponding to the second collimating surface 141 onto the detector, resulting in better imaging quality for examining important parts of the test subject. In this way, the CT machine in which the collimator of some embodiments of the present disclosure is configured may achieve good diagnostic performance while maintaining a compact structure.

In some embodiments, the collimator body 100 may further include a third collimating surface 132 and a fourth collimating surface 142. The third collimating surface 132 may be arranged corresponding to the first collimating surface 131. The third collimating surface 132 and the first collimating surface 131 may be arranged on two opposite sides of the collimator body 100, respectively. That is, in a case where the first collimating surface 131 is arranged on the side of the collimator body 100 facing the X-ray tube, the third collimating surface 132 may be arranged on the side of the collimator body 100 facing away from the X-ray tube. The curvature of the first collimating surface 131 may be substantially equal to a curvature of the third collimating surface 132, enabling the first collimating surface 131 and the third collimating surface 132 to be substantially parallel to each other. A part of the collimator body 100 between the first collimating surface 131 and the third collimating surface 132 may have a completely curved structure. In this way, a shape of a projection of the X-rays passing a part of the ray channel 150 corresponding to both the first collimating surface 131 and the third collimating surface 132 onto the detector may be relatively similar to a rectangle, thereby improving imaging quality of the images created by the X-rays.

The fourth collimating surface 142 may be arranged corresponding to the second collimating surface 141. The fourth collimating surface 142 and the second collimating surface 141 may be arranged on two opposite sides of the collimator body 100, respectively. That is, in a case where the second collimating surface 141 is arranged on the side of the collimator body 100 facing the X-ray tube, the fourth collimating surface 142 may be arranged on the side of the collimator body 100 facing away from the X-ray tube. The curvature of the second collimating surface 141 may be substantially equal to a curvature of the fourth collimating surface 142, enabling the second collimating surface 141 and the fourth collimating surface 142 to be substantially parallel to each other. In this way, a shape of a projection of the X-rays passing a part of the ray channel 150 corresponding to both the second collimating surface 141 and the fourth collimating surface 142 on the detector may be relatively similar to a rectangle, thereby improving imaging quality of the images created by the X-rays.

In some embodiments, the first collimating surface 131 of the collimator body 100 and the second collimating surface 141 of the collimator body 100 may be connected to each other, enabling the first collimating surface 131 and the second collimating surface 141 to form a continuous structure. Correspondingly, the ray channel 150 of the collimator body 100 may extend from the first collimating surface 131 to the second collimating surface 141, thereby enabling the ray channel 150 to have a continuous structure. In this way, the X-rays emitted from the X-ray tube may remain continuous after passing through the collimator body 100, thereby allowing the X-rays to fully pass through the test subject's body and resulting in more complete diagnostic results.

In some embodiments, to further standardize the structure of the collimator body 100 of the present disclosure, an area of the first collimating surface 131 of the collimator body 100 may set to be substantially equal to an area of the third collimating surface 132 of the collimator body 100, thereby enabling a relatively standardized curved structure between the first collimating surface 131 of the collimator body 100 and the third collimating surface 132 of the collimator body 100. An area of the second collimating surface 141 of the collimator body 100 may further be set to be substantially equal to an area of the fourth collimating surface 142 of the collimator body 100, thereby further enabling a relatively standardized structure between the second collimating surface 141 of the collimator body 100 and the fourth collimating surface 142 of the collimator body 100.

In some embodiments, to further enable the compact structure of the collimator of the present disclosure, each of the second collimating surface 141 of the collimator body 100 and the fourth collimating surface 142 of the collimator body 100 may set to be a flat surface, allowing a thickness of each part of the collimator body 100 between the second collimating surface 141 and the fourth collimating surface 142 to be substantially uniform. As a result, the part of the collimator body 100 corresponding to both the second collimating surface 141 and the fourth collimating surface 142 may occupy relatively small space in the Y-axis direction of the CT machine.

In some embodiments, the part of the collimator body 100 corresponding to both the second collimating surface 141 and the fourth collimating surface 142 may set to have a flat plate structure, which may simplify the structure of such part of the collimator and reduce manufacturing difficulty of the collimator body 100. A part of the collimator body 100 corresponding to the first collimating surface 131 may be manufactured separately from a part of the collimator body 100 corresponding to the second collimating surface 141. In other words, the curved structure of the collimator body 100 and the flat structure of the collimator body 100 may be manufactured separately from each other and then fixed together through a welding process or the like, thereby further reducing the manufacturing difficulty of the collimator of some embodiments of the present disclosure. In some embodiments, the collimator body 100 may be integrally formed, which may enhance the structural stability of the collimator body 100.

In some embodiments, in order to broaden a detection range of the CT machine in which the collimator of the present disclosure is configured, the number of the second collimating surface 141 may be set to two. The number of the fourth collimating surface 142 may be set to two. The two second collimating surfaces 141 may be arranged on two sides of the first collimating surface 131, respectively. In some embodiments, the part of the collimator body 100 corresponding to both the first collimating surface 131 and the third collimating surface 132 may be defined as a first collimating section 130. The part of the collimator body 100 corresponding to both the second collimating surface 141 and the fourth collimating surface 142 may be defined as a second collimating section 140. That is, the number of the second collimating section 140 of the collimator body 100 may be two. The two second collimating sections 140 may be disposed on two opposite sides of the first collimating section 130, respectively. It may be understood that, the main organs of the human body may be located in the central part of the body and other parts such as the hands and feet, etc., may be located on the two sides of the human body. In this way, in a case where detection is performed using the CT machine according to some embodiments of the present disclosure, the important organ regions to be detected may be positioned corresponding to the first collimating section 130. The hands and feet, etc., to be detected, which have a lower image quality requirement, may be positioned corresponding to the second collimating sections 140 respectively disposed on two sides of the first collimating section 130. Accordingly, both hands and feet of the test subject may be detected. In addition, a proportion of the first collimating section 130 in the collimator body 100 may be reduced and a proportion of the second collimating sections 140 in the collimator body 100 may be increased, thereby enabling the compact structure of the collimator and enabling the collimator to occupy relatively small internal space of the CT machine.

It may be understood that the ray channel 150 of the collimator body 100 may extend from one of the two second collimating sections 140 on one side, through the first collimating section 130, to the other one of the second collimating sections 140 on the other side. In this way, a range of the ray channel 150 may be relatively large, further enabling a great amount of X-rays to pass through the ray channel 150.

In some embodiments, in order to reduce the difficulty of manufacturing the collimator of the present disclosure, the collimator body 100 may set to include a first collimating portion 110 and a second collimating portion 120. The first collimating portion 110 and the second collimating portion 120 may be spaced apart from and in opposite to each other, enabling the ray channel 150 to be formed between the first collimating portion 110 and the second collimating portion 120. It may be understood that each of the first collimating surface 131, the second collimating surface 141, the third collimating surface 132, and the fourth collimating surface 142 of the collimator body 100 may be arranged on both the first collimating portion 110 and the second collimating portion 120. In this way, the ray channel 150 defined between the first collimating portion 110 and the second collimating portion 120 may be enabled to correspond to each of the first collimating surface 131, the second collimating surface 141, the third collimating surface 132, and the fourth collimating surface 142. Through forming or defining the ray channel 150 between the first collimating portion 110 and the second collimating portion 120, it may not be necessary to provide a through-hole structure in the collimator body 100, thereby simplifying the manufacturing operations of the collimator body 100 and minimizing material cost.

A structure of the first collimating portion 110 may be substantially the same as a structure the second collimating portion 120, enabling the first collimating portion 110 and the second collimating portion 120 to be arranged symmetrically along the ray channel 150. During the manufacturing of the collimator according to some embodiments of the present disclosure, the first collimating portion 110 and the second collimating portion 120 may be manufactured in batches using the same process, thereby reducing the manufacturing cost of the collimator of some embodiments of the present disclosure.

In some embodiments, in order to improve the adaptability of the CT machine in which the collimator of the present disclosure is configured in, an opening size of the ray channel 150 of the collimator body 100 may be made adjustable. In this way, the amount of X-rays passing through the ray channel 150 of the collimator body 100 may be adjusted, thereby achieving the purpose of adjusting a slice thickness of the X-rays. In some embodiments, a distance between the first collimating portion 110 and the second collimating portion 120 may be adjustable, enabling a size of the ray channel 150 between the first collimating portion 110 and the second collimating portion 120 to be adjustable. In a case where the first collimating portion 110 and the second collimating portion 120 are relatively close to each other, the size of the ray channel 150 may be reduced, thereby reducing the slice thickness of the X-rays. In a case where the first collimating portion 110 and the second collimating portion 120 are relatively far apart from each other, the size of the ray channel 150 may be increased, thereby increasing the slice thickness of the X-rays. In this way, the distance between the first collimating portion 110 and the second collimating portion 120 may be flexibly adjusted according to different detection schemes, enabling the size of the ray channel 150 to be adapted to various detection schemes.

In some embodiments, in a case where the collimator of the present disclosure is configured in the CT machine, a driving device may be disposed in the CT machine. The driving device may be configured to control the distance between the first collimating portion 110 and the second collimating portion 120. The driving device may be connected to each of the first collimating portion 110 and the second collimating portion 120, enabling both the first collimating portion 110 and the second collimating portion 120 to be fixed inside the CT machine. In some embodiments, the driving device may include a driving motor.

In some embodiments, the second collimating surface 141 of the collimator body 100 may have one end connected to the first collimating surface 131 and another end away from the first collimating surface 131. A direction from the one end of the second collimating surface 141 to the another end of the second collimating surface 141 may be defined as a second direction. The part of the first collimating portion 110 corresponding to both the second collimating surface 141 and the fourth collimating surface 142 may include a first sidewall 111 arranged on one side of a first direction and a second sidewall 112 arranged on another side of the first direction. A distance between the first sidewall 111 of the first collimating portion 110 and the second sidewall 112 of the first collimating portion 110 may gradually increase in the first direction, enabling the second collimating section 140 of the first collimating portion 110 to have an outwardly expanding structure. It may be understood that in a case where the distance between the first sidewall 111 of the first collimating portion 110 and the second sidewall 112 of the first collimating portion 110 remains unchanged in the first direction and a distance between the first sidewall 111 of the second collimating portion 120 and the second sidewall 112 of the second collimating portion 120 remains unchanged in the first direction, then a part of the ray channel 150 formed between the second collimating section 140 of the first collimating portion 110 and the second collimating section 140 of the second collimating portion 120 may be a rectangular channel. In such a case, a projection of the X-rays emitted from the X-ray tube passing through the part of the ray channel 150, between the second collimating section 140 of the first collimating portion 110 and the second collimating section 140 of the second collimating portion 120, on the detector may be substantially spindle-shaped, resulting in poor detection performance for such part of the ray channel 150.

In some embodiments of the present disclosure, the second collimating section 140 of the first collimating portion 110 and the second collimating section 140 of the second collimating portion 120 may each be configured to have the outwardly expanding structure, such that the ray channel 150 formed between the second collimating section 140 of the first collimating portion 110 and the second collimating section 140 of the second collimating portion 120 may have a non-rectangular structure. The above arrangement may correct a shape of the projection of the X-rays through the part of the ray channel 150 between the second collimating section 140 of the first collimating portion 110 and the second collimating section 140 of the second collimating portion 120 on the detector to a certain extent, enabling the shape of the projection to be relatively similar to a rectangle.

Each of the first sidewall 111 of the first collimating portion 110, the second sidewall 112 of the first collimating portion 110, the first sidewall 111 of the second collimating portion 120, and the second sidewall 112 of the second collimating portion 120 may have a curved surface. In this way, in a case where both a position of the X-ray tube and a parameter of the emitted X-rays are determined, a curved surface size of each of the first sidewall 111 and the second sidewall 112 may be designed such that the shape of the projection of the X-rays through the part of the ray channel 150 between the second collimating section 140 of the first collimating portion 110 and the second collimating section 140 of the second collimating portion 120 on the detector may be corrected into substantially a rectangle. In some embodiments, the size of the first sidewall 111 and the size of the second sidewall 112 may each be calculated according to the following formula:

$$z = \frac{R_{fc}}{R_{fd} \cdot \cos(\beta)} \cdot z_0$$

In the above formula, $z_0$ may represent a size of the detector in a row direction, i.e., a Z-direction of the CT machine, $R_{fd}$ may represent a distance from a focal spot of the X-rays emitted by the X-ray tube to the detector, $R_{fc}$ may represent a distance from the focal spot of the X-rays emitted by the X-ray tube to the second collimating section 140, and $\beta$ may represent an angle between a center line of the collimator body 100 and a connecting line between any pair of opposite points on the first sidewall 111 and the second sidewall 112 and the focal spot of the X-rays emitted by the X-ray tube. A distance between each pair of opposite points on the first sidewall 111 and the second sidewall 112 may be obtained according to this formula, such that a structural dimension of the second collimating section 140 may be designed based on this formula.

Some embodiments of the present disclosure may further provide a CT machine. The CT machine may include the collimator as described above.

In some embodiments of the present disclosure, the curvature of the first collimating surface in the collimator body and the curvature of the second collimating surface in the collimator body may be different from each other. The curvature of the first collimating surface may be greater than the curvature of the second collimating surface, enabling the part of the collimator body corresponding to the second collimating surface to occupy a smaller space than the part of the collimator body corresponding to the first collimating surface. In this way, the structure of the collimator may be relatively compact, and the space occupied by the collimator in the CT machine in which the collimator of some embodiments of the present disclosure is configured in may be reduced.

The shape of the projection of the X-rays through the part of the ray channel corresponding to the first collimating surface on the detector may be relatively similar to a rectangle, enabling an image corresponding to the part of the ray channel to have better imaging performance. The above image may be used for detecting important detection parts of the test subject, thereby enabling the CT machine in which the collimator of some embodiments the present disclosure is configured in to achieve good detection performance while maintaining a relatively compact structure.

In the description of the present specification, references to the terms "one embodiment," "some embodiments," "example," "specific example," or "some examples" may mean that the specific features, structures, materials, or characteristics described in connection with the embodiment or example may be included in at least one embodiment or example of the present disclosure. In the present specification, the schematic representation of the above terms may not necessarily refer to the same embodiment or example. Moreover, the specific features, structures, materials, or characteristics described may be combined in any suitable manner in one or more embodiments or examples. In addition, those skilled in the art may combine and integrate the different embodiments or examples described in the present specification.

**Claims**

1. A collimator, comprising:
   a collimator body (100), comprising a first collimating surface (131) facing or facing away from an X-ray tube and a second collimating surface (141) facing or facing away from the X-ray tube, wherein:

   a curvature of the first collimating surface (131) is greater than a curvature of the second collimating surface (141), and the first collimating surface (131) and the second collimating surface (141) are arranged in a staggered manner; and
   the collimator body (100) defines a ray channel (150) penetrating through the collimator body (100), and the ray channel (150) is defined by the first collimating surface (131) and the second collimating surface (141).

2. The collimator as claimed in claim 1, wherein:

   the collimator body (100) further comprises a third collimating surface (132) and a fourth collimating surface (142);
   the third collimating surface (132) is arranged on one side of the collimator body (100), and the first collimating surface (131) is arranged on another side of the collimator body (100) in opposite to the third collimating surface (132) and is arranged corresponding to the third collimating surface (132);

the fourth collimating surface (142) is arranged on one side of the collimator body (100), and the second collimating surface (141) is arranged on another side of the collimator body (100) in opposite to the fourth collimating surface (142) and is arranged corresponding to the fourth collimating surface (142); and

the curvature of the first collimating surface (131) is equal to a curvature of the third collimating surface (132), and the curvature of the second collimating surface (141) is equal to a curvature of the fourth collimating surface (142).

3. The collimator as claimed in claim 2, wherein an area of the first collimating surface (131) is equal to an area of the third collimating surface (132), and an area of the second collimating surface (141) is equal to an area of the fourth collimating surface (142).

4. The collimator as claimed in claim 3, wherein each of the second collimating surface (141) and the fourth collimating surface (142) is a flat surface.

5. The collimator as claimed in claim 4, wherein a direction from the first collimating surface (131) to the second collimating surface (141) is a first direction, and a shape of a projection of the first collimating surface (131) on a predetermined plane in the first direction is a rectangle.

6. The collimator as claimed in claim 5, wherein the first collimating surface (131) is connected to the second collimating surface (141), the third collimating surface (132) is connected to the fourth collimating surface (142), and the ray channel (150) extends from the first collimating surface (131) to the second collimating surface (141).

7. The collimator as claimed in claim 6, wherein:

the number of the second collimating surfaces (141) is two, the number of the fourth collimating surfaces (142) is two; and

the two second collimating surfaces (141) and the two fourth collimating surfaces (142) are arranged on two sides of the first collimating surface (131).

8. The collimator as claimed in any one of claims 1-7, wherein the collimator body (100) comprises a first collimating portion (110) and a second collimating portion (120), the first collimating portion (110) and the second collimating portion (120) are spaced apart from and in opposite to each other, and the ray channel (150) is defined between the first collimating portion (110) and the second collimating portion (120).

9. The collimator as claimed in claim 8, wherein a distance between the first collimating portion (110) and the second collimating surface (141) is adjustable.

10. The collimator as claimed in claim 9, wherein a structure of the first collimating portion (110) is the same as a structure of the second collimating portion (120).

11. The collimator as claimed in claim 10, wherein:

a direction from an end of the second collimating surface (141) connected to the first collimating surface (131) to another end of the second collimating surface (141) facing away from the first collimating surface (131) is a second direction;

a part of the first collimating portion (110) corresponding to the second collimating surface (141) comprises a first sidewall (111) arranged on one side of the second direction and a second sidewall (112) arranged on another side of the second direction; and

in the second direction, a distance between the first sidewall (111) and the second sidewall (112) gradually increases.

12. The collimator as claimed in claim 11, wherein each of the first sidewall (111) and the second sidewall (112) is a curved surface.

13. A CT machine, comprising the collimator as claimed in any one of claims 1-12.

FIG. 1

FIG. 2

FIG. 3

## INTERNATIONAL SEARCH REPORT

| International application No. |
|---|
| **PCT/CN2024/073625** |

| A. | CLASSIFICATION OF SUBJECT MATTER |
|---|---|

A61B6/06(2006.01)i; A61B6/03(2006.01)i

According to International Patent Classification (IPC) or to both national classification and IPC

| B. | FIELDS SEARCHED |
|---|---|

Minimum documentation searched (classification system followed by classification symbols)

IPC: A61B6

Documentation searched other than minimum documentation to the extent that such documents are included in the fields searched

Electronic data base consulted during the international search (name of data base and, where practicable, search terms used)

CNTXT, ENTXTC, VEN, DWPI, WPABS: 美的, 准直器, 弧, 曲率, 矩形, 投影, collimator, radian, arc+, curvature, rectangular, project+

| C. | DOCUMENTS CONSIDERED TO BE RELEVANT |
|---|---|

| Category* | Citation of document, with indication, where appropriate, of the relevant passages | Relevant to claim No. |
|---|---|---|
| X | CN 103829963 A (GE MEDICAL SYSTEMS GLOBAL TECHNOLOGY COMPANY, LLC.) 04 June 2014 (2014-06-04) description, paragraphs 24-54, and figures 1A-2D | 1-10, 13 |
| Y | CN 103829963 A (GE MEDICAL SYSTEMS GLOBAL TECHNOLOGY COMPANY, LLC) 04 June 2014 (2014-06-04) description, paragraphs 24-54, and figures 1A-2D | 11-13 |
| Y | CN 107582089 A (SHANGHAI UNITED IMAGING HEALTHCARE CO., LTD.) 16 January 2018 (2018-01-16) description, paragraphs 74-77, and figure 4A | 11-13 |
| X | CN 1681437 A (SIEMENS AG) 12 October 2005 (2005-10-12) description, pages 4-8, and figures 1, 3-7 | 1-10, 13 |
| X | CN 111759332 A (SINOVISION TECHNOLOGIES (BEIJING) CO., LTD.) 13 October 2020 (2020-10-13) description, paragraphs 35-64, and figures 1-2 | 1-10, 13 |
| X | US 2020051708 A1 (GENERAL ELECTRIC COMPANY) 13 February 2020 (2020-02-13) description, paragraphs 24-31 and 56-59, and figures 1-2 and 10-12 | 1-10, 13 |

| ✓ Further documents are listed in the continuation of Box C. | ✓ See patent family annex. |
|---|---|

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **28 April 2024** | **29 April 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2024/073625** |

**C.      DOCUMENTS CONSIDERED TO BE RELEVANT**

| Category* | Citation of document, with indication, where appropriate, of the relevant passages | Relevant to claim No. |
| --- | --- | --- |
| A | CN 103961131 A (SINOVISION TECHNOLOGIES (BEIJING) CO., LTD.) 06 August 2014 (2014-08-06)<br>    entire document | 1-13 |
| A | CN 212847714 U (NANJING ANKE MEDICAL TECHNOLOGY CO., LTD.) 30 March 2021 (2021-03-30)<br>    entire document | 1-13 |
| A | US 2002015474 A1 (ANALOGIC CORPORATION) 07 February 2002 (2002-02-07)<br>    entire document | 1-13 |
| A | JP 2002034966 A (SHIMADZU CORPORATION) 05 February 2002 (2002-02-05)<br>    entire document | 1-13 |
| A | US 2012307963 A1 (WATANABE KUNIO et al.) 06 December 2012 (2012-12-06)<br>    entire document | 1-13 |

Form PCT/ISA/210 (second sheet) (July 2022)

## INTERNATIONAL SEARCH REPORT
### Information on patent family members

International application No.

**PCT/CN2024/073625**

| Patent document cited in search report | | | Publication date (day/month/year) | Patent family member(s) | | | Publication date (day/month/year) |
|---|---|---|---|---|---|---|---|
| CN | 103829963 | A | 04 June 2014 | JP | 2014104355 | A | 09 June 2014 |
| | | | | JP | 6334898 | B2 | 30 May 2018 |
| | | | | US | 2014146949 | A1 | 29 May 2014 |
| | | | | US | 9237875 | B2 | 19 January 2016 |
| CN | 107582089 | A | 16 January 2018 | US | 2022401058 | A1 | 22 December 2022 |
| | | | | US | 2019099149 | A1 | 04 April 2019 |
| | | | | US | 11419572 | B2 | 23 August 2022 |
| CN | 1681437 | A | 12 October 2005 | JP | 2005538786 | A | 22 December 2005 |
| | | | | US | 2006050841 | A1 | 09 March 2006 |
| | | | | US | 7170975 | B2 | 30 January 2007 |
| | | | | WO | 2004026141 | A1 | 01 April 2004 |
| | | | | DE | 10242920 | A1 | 25 March 2004 |
| | | | | DE | 10242920 | B4 | 22 August 2013 |
| CN | 111759332 | A | 13 October 2020 | None | | | |
| US | 2020051708 | A1 | 13 February 2020 | US | 10695011 | B2 | 30 June 2020 |
| CN | 103961131 | A | 06 August 2014 | None | | | |
| CN | 212847714 | U | 30 March 2021 | None | | | |
| US | 2002015474 | A1 | 07 February 2002 | US | 6396902 | B2 | 28 May 2002 |
| JP | 2002034966 | A | 05 February 2002 | JP | 3562449 | B2 | 08 September 2004 |
| US | 2012307963 | A1 | 06 December 2012 | WO | 2012093695 | A1 | 12 July 2012 |
| | | | | JP | 2012152550 | A | 16 August 2012 |
| | | | | EP | 2662023 | A1 | 13 November 2013 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310144907